# EUROPEAN PATENT SPECIFICATION

(11) **EP 2 774 623 B1**
(45) Date of publication and mention of the grant of the patent: **08.04.2015**
(21) Application number: 14171384.2
(22) Date of filing: 31.05.2005
(51) Int. Cl.: A61K 45/06, A61K 31/573, A61K 31/58, A61K 31/407, A61K 31/439, A61K 31/4439, A61K 31/167, A61K 31/137, A61K 31/655, A61P 11/00, A61K 9/00, A61P 11/06

(54) **Combinations comprising antimuscarinic agents and beta-adrenergic agonists**
Zusammensetzungen mit Antimuskarinwirkstoffen und beta-adrenergischen Agonisten
Combinaisons comportant des agents anti-muscariniques et agonistes bêta-adrénergiques

(30) Priority: 31.05.2004 ES 200401312
(43) Date of publication of application: 10.09.2014
(62) Divisional of application: 10010080.9
(73) Proprietor: Almirall S.A., 08022 Barcelona (ES)
(72) Inventor: Gras Escardo, Jordi, 08018 Barcelona (ES); Llenas Calvo, Jesús, 08021 Barcelona (ES); Ryder, Hamish, Cambridge, Cambridgeshire CB2 8DZ (GB); Orviz Díaz, Pío, 08980 Sant Feliu de Lobregat Barcelona (ES)
(74) Representative: Srinivasan, Ravi Chandran

(56) References cited:
- WO-A-01/04118
- WO-A-02/38154
- WO-A-02/053564
- WO-A-03/000241
- WO-A-03/087094
- WO-A1-2005/014044

## Description

The present invention relates to new combinations of certain antimuscarinic agents with β-adrenergic agonists and their use in the treatment of respiratory disorders.

### BACKGROUND OF THE INVENTION

β-adrenergic agonists, In particular β2-adrenergic agonists, and antimuscarinic agents, in particular antagonists of M3 muscarinic receptors, are two classes of bronchodilating drugs useful in the treatment of respiratory disorders, such as asthma or Chronic Obstructive Respiratory Diseases (COPD).

It is known that both classes of drugs can be used in combination. The International Patent Applications WO0238154 and WO03000241 describe some examples of such combinations.

Combinations of drugs in which the active ingredients operate via different physiological pathways are known to be therapeutically useful. Frequently, the therapeutic advantage arises because the combination can achieve a therapeutically useful effect using lower concentrations of each active component. This enables the side-effects of the medication to be minimised. Thus, the combination can be formulated so that each active ingredient is present at a concentration which is subclinical in cells other than the target disease cells. The combination is nevertheless therapeutically effective in target cells which respond to both ingredients.

Notwithstanding the above discussion, combinations of known M3 muscarinic receptors and β-adrenergic agonists which are used in combination to treat respiratory disorders, are known to have an unwanted effect in the heart. Cardiac cells appear to be susceptible both to known M3 antagonists and to β-adrenergic agonists in the same way as cells in the respiratory tract. The cardiac side effects appear to be more prominent and frequent when both classes of drugs are used in combination. Thus, the use of combinations of known antimuscarinic agents and β-adrenergic agonists involve undesirable cardiac side-effects e.g. tachycardia, palpitations, angina-like complaints and arrhythmias, limiting thus the therapeutic value, of the combination, especially in patients with an underlying heart condition.

### DESCRIPTION OF THE INVENTION

Surprisingly, it has now been found that a combination of certain specific antagonists of M3 muscarinic receptors (further on referred to as the M3 antagonists of the invention) with β2-adrenergic agonists (further on referred to as β2-agonists) produce significantly less heart side-effects, such as tachycardia, than the combinations proposed in the art, yet retaining a robust activity in the respiratory tract.

The present invention accordingly provides a combination which comprises (a) a β2 agonist and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

Examples of pharmaceutically acceptable anions of mono or polyvalent acids are the anions derived from inorganic acids such as hydrochloric acid, hydrobromic acid, sulphuric acid, phosphoric acid or organic acids such as methanosulphonic acid, acetic acid, fumaric acid, succinic acid, lactic acid, citric acid or maleic acid. Furthermore, mixtures of the aforementioned acids can be used.

Typically, the antagonist of M3 muscarinic receptors is 3(R)-(2-Hydroxy-2,2-dithien-2-ylacetoxy)-1-(3-phenoxypropyl)-azoniabicyclo[2.2.2]octane bromide. Typically the combination contains the active ingredients (a) and (b) forming part of a single pharmaceutical composition.

For the avoidance of doubt, the formula depicted above and the term 3(R)-(2-hydroxy-2,2-dithien-2-ylacetoxy)-1-(3-phenoxypropyl)-1-azoniabicyclo[2.2.2]octane is meant to embrace the salts in dissociated, partially dissociated or undissociated form, for example in aqueous solution. The different salts of the compound may exist in the form of solvates, i.e. in the form of hydrates and all these forms are also within the scope of the present invention. Furthermore the different salts and solvates of the compound may exist in amorphous form or in the form of different polymorphs within the scope of the present invention.

Also provided is a product comprising (a) a β2-agonist and (b) an M3 antagonist of the invention as a combined preparation for simultaneous, separate or sequential use in the treatment of a human or animal patient. Typically the product is for simultaneous, separate or sequential use in the treatment of a respiratory disease which is asthma, acute or chronic bronchitis, emphysema, chronic obstructive pulmonary disease (COPD), bronchial hyperreactivity or rhinitis, in a human or animal patient

The present invention further provides the use of (a) a β2-agonist, and (b) an M3 antagonist of the invention for the preparation of a medicament for simultaneous, concurrent, separate or sequential use in the treatment of a said respiratory disease in a human or animal patient.

Also provided is the use of (b) an M3 antagonist of the invention for the preparation of a medicament, for simultaneous, concurrent, separate or sequential use in combination with (a) a β2 agonist for the treatment of a said respiratory disease in a human or animal patient.

Also provided is the use of (a) a β2-agonist for the preparation of a medicament for use in the treatment of a said respiratory disease in a human or animal patient by simultaneous, concurrent, separate or sequential co-administration with (b) an M3 antagonist of the invention.

Typically, said respiratory disease is asthma or chronic obstructive pulmonary disease (COPD).

Typically the said human or animal patient is suffering from a pre-existing heart condition or a condition that would be aggravated by tachycardia, e.g., patients having pre-existing cardiac arrhythmia, hypo- or hypertension, angina or angina-like complaints, history of myocardial infarction, coronary artery disease or elderly patients. Preferably said patient is human.

Also provided is a pharmaceutical composition comprising (a) a β2-agonist and (b) an M3 antagonist of the invention in association with (c) a pharmaceutically acceptable carrier or diluent.

The invention also provides a kit of parts comprising (b) an M3 antagonist of the invention together with instructions for simultaneous, concurrent, separate or sequential use in combination with (a) a β2 agonist, for the treatment of a human or animal patient suffering from or susceptible to a said respiratory disease.

Further provided is a package comprising (b) an M3 antagonist of the invention and (a) a β2 agonist for the simultaneous, concurrent, separate or sequential use in the treatment of a said respiratory disease.

Further provided is a combination, product, kit of parts or package as hereinabove described wherein such combination, product, kit of parts or package further comprises (c) another active compound selected from: (a) PDE IV inhibitors, (b) cortiocosteroids, (c) leukotriene D4 antagonists, (d) inhibitors of egfr-kinase, (e) p38 kinase inhibitors and (f) NK1 receptor agonists for simultaneous, separate or sequential use. Typically the additional active compound (c) is selected from the group consisting of (a) PDE IV inhibitors and (b) cortiocosteroids.

It is a embodiment of the present invention that the combination, product, kit of parts or package comprises (b) an M3 antagonist of the invention and (a) a β2 agonist, as the sole active compounds.

It is also an embodiment of the present invention the use of b) an M3 antagonist of the invention and (a) a β2 agonist without any other active compound for the preparation of a medicament for simultaneous, concurrent, separate or sequential use in the treatment of a respiratory disease which responds to M3 antagonism in a human or animal patient.

The preferred β2-agonists to be used in the combinations of the invention are: arformoterol, bambuterol, bitolterol, broxaterol, carbuterol, clenbuterol, dopexamine, fenoterol, formoterol, hexoprenaline, ibuterol, isoetharine, isoprenaline, levosalbutamol, mabuterol, meluadrine, metaprotenerol, nolomirole, orciprenaline, pirbuterol, procaterol, reproterol, ritodrine; rimoterol, salbutamol, salmefamol, salmeterol, sibenadet, sotenerot, sulfonterol, terbutaline, tiaramide, tulobuterol, GSK-597901, GSK-159797, GSK-678007, GSK-642444, GSK-159802, HOKU-81, (-)-2-[7(S)-[2(R)-Hydroxy-2-(4-hydroxyphenyl)ethylamino]-5,6,7,8 tatrahydro-2-naphthyloxy]-N,N-dimethylacetamide hydrochloride monohydrate, carmoterol, QAB-149 and 5-[2-(5,6-diethylindan-2-ylamino)-1-hydroxyethyl]-8-hydroxy;-1H-quinolin-2-one, 4-hydroxy-7-[2-{[2-{[3-(2-phenylethoxy)propyl]sulfonyl} ethyl]amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propyiamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tert-butylamino)ethanol and 1-(4-ethoxycarbonylamino-3-cyano-5-fluorophenyl)-2-(tert-butylamino)ethanol optionally in the form of their racemates, their enantiomers, their diastereomers, and mixtures thereof, and optionally their pharmacologically-compatible acid addition salts.

The preferred β2-agonists to be used in the combinations of the invention are: arformoterol, bambuterol, bitolterol, broxaterol, carbuterol, clenbuterol, dopexamine, fenoterol, formoterol, hexoprenaline, ibuterol, isoprenaline, levosalbutamol, mabuterol, meluadrine, nolomirole, orciprenaline, pirbuterol, procaterol, (R,R)-formoterol, reproterol, ritodrine, rimoterol, salbutamol, salmeterol, sibenadet, sulfonterol, terbutaline, tulobuterol, GSK-597901, GSK-159797, KUL-1248, TA-2005 and QAB-149 optionally In the form of their racemates, their enantiomers, their diastereomers, and mixtures thereof, and optionally their pharmacologically-compatible acid addition salts.

Since the M3 antagonists of the invention have a long duration of action, it is preferred that they are combined with long-acting β2-agonists (also known as LABAs). The combined drugs could thus be administered once a day.

Particularly preferred LABAs are formoterol, salmeterol and GSK-597901, GSK-159797, KUL-1248, TA-2005 and QAB-149 optionally in the form of their racemates, their enantiomers, their diastereomers and mixtures thereof, and optionally their pharmacologically compatible acid addition salts. More preferred are salmeterol, formoterol and QAB-149. Still more preferred are salmeterol and formoterol, In particular salmeterol xinafoate and formoterol fumarate.

The following can be considered to represent examples of suitable acid for the formation of addition salts of the β2-agonists: hydrochloric acid, hydrobromic acid, sulphuric acid, phosphoric acid, methanosulphonic acid, acetic acid, fumaric acid, succinic acid, lactic acid, citric acid, maleic acid; and trifluoroacetic acid. Furthermore, mixtures of the aforementioned acids can be used.

A preferred embodiment of the present invention is a combination of an M3 antagonist of the invention with a LABA selected from formoterol, salmeterol, GSK-597901, GSK-159797, KUL-1248, TA-2005 and QAB-149.

A particularly preferred embodiment of the present invention is a combination of an M3 antagonist of the invention with a LABA selected from formoterol, salmeterol, GSK-597901, GSK-159797, KUL-1248, TA-2005 and QAB-149.

Another embodiment of the present invention is a combination of an M3 antagonist which is 3(R)-(2-Hydroxy-2,2-dithlen-2-ylacetoxy)-1-(3-phenoxypropyl)-1-azoniabicyclo[2.2.2]octane bromide, with a LABA selected from formoterol, salmeterol, GSK-597901, GSK-159797, KUL-1248, TA-2005 and QAB-149.

According to one embodiment of the invention the β2-agonist is formoterol, in particular formoterol fumarate.

According to another embodiment of the invention the β2-agonist is salmeterol, in particular salmeterol xinafoate.

The combinations of the invention can optionally comprise one or more additional active substances which are known to be useful in the treatment of respiratory disorders, such as PDE4 inhibitors, corticosteroids or glucocorticoids, leukotriene D4 inhibitors, inhibitors of egfr-kinase, p38 kinase inhibitors and/or NK1-receptor antagonists.

Examples of suitable PDE4 inhibitors that can be combined with M3-antagonists and β2-agonists are denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide, 9-(2-Fluorobenzyl)-N6-methyl-2-(trifluoromethyl)adenine, N-(3,5-Dichloro-4-pyridinyl)-8-mothoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)py(idin-2(1H)-one, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/097613 and PCT/EP03/14722 and in the Spanish patent application number P200302613.

Examples of suitable corticosteroids and glucocorticoids that can be combined with M3-antagonists and β2-agonists are prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone famesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable LTD4 antagonists that can be combined with M3 antagonists and β2-agonists are tomelukast, Ibudilast, pobilukast, praniukast hydrate, zafirlukast, ritolukast, verlukast, sulukast, cinalukast, Iralukast sodium, montelukast sodium, 4-[4-[3-(4-Acetyl-3-hydroxy-2-propylphenoxy)propylsulfonyl]phenyl]-4-oxobutyric acid, [[5-[[3-(4-Acetyl-3-hydroxy-2-propylphenoxy)propyl]thio]-1,3,4-thiadiazol-2-yl]thio]acetic acid, 9-[(4-Acetyl-3-hydroxy-2-n-propylphenoxy)methyl]-3-(1H-tetrazol-5-yl)-4H-pyrido[1,2-a]pyrimidin-4-one, 5-[3-[2-(7-Chloroquinolin-2-yl)vinyl]phenyl]-B-(N,N-dimethylcarbamoyl)-4,6-dithiaoctanoic acid sodium salt; 3-[1-[3-[2-(7-Chloroquinolin-2-yl)vinyl]phenyl]-1-[3-(dimethylamino)-3-oxopropylsulfanyl]methylsulfanyl]propionic acid sodium salt, 6-(2-Cyclohexylethyl)-[1,3,4]thiadiazolo[3,2-a]-1,2,3-triazolo[4,5-d]pyrimidin-9(1H)-one, 4-[6-Acetyl-3-[3-(4-acetyl-3-hydroxy-2-propylphenylthio)propoxy]-2-propylphenoxy]butyric acid, (R)-3-Methoxy-4-[1-methyl-5-[N-(2-methyl-4,4,4-trifluorobutyl)carbamoyl]indol-3-ylmethyl]-N-(2-methylphenylsulfonyl)benzamide, (R)-3-[2-Methoxy-4-[N-(2-methylphenylsulfonyl)carbamoyl]benzyl]-1-methyl-N-(4,4,4-trifluoro-2-methylbutyl)indofe-5-carboxamide, (+)-4(S)-(4-carboxyphenylthio)-7-[4-(4-phenoxybutoxy)phenyl]-5(Z)-heptenoic acid and the compounds claimed in the PCT patent application number PCT/EP03/12581.

Examples of suitable inhibitors of egfr-kinase that can be combined with M3 antagonists and β2-agonists are palifermin, cetuximab, gefitinib, repifermin, erlotinib hydrochloride, canertinib dihydrochloride, lapatinib, and N-[4-(3-Chloro-4-fluorophenylamino)-3-cyano-7-ethoxyquinolin-6-yl]-4-(dimethylamino)-2(E)-butenamide.

Examples of suitable p38-kinase inhibitors that can be combined with M3 antagonists and β2-agonists are chlormethiazole edisylate, doramapimod, 5-(2,6-Dichlorophenyl)-2-(2,4-difluorophenylsulfanyl)-6H-pyrimido[3,4-b]pyridazin-6-one, 4-Acetamido-N-(tert-butyl)benzamide, SCIO-469 (described in Clin Pharmacol Ther 2004, 75(2): Abst PII-7 and VX-702 described in Circulation 2003, 108(17, Suppl. 4): Abst 882.

Examples of suitable NK1-receptor antagonists that can be combined with M3 antagonists and β2-agonlsts are nolpitantium besilate, dapitant, lanepitant, vofopitant hydrochloride, aprepitant, ezlopitant, N-[3-(2-Pentylphenyl)propionyl]-threonyl-N-methyl-2,3-dehydrotyrosyl-leucyl-D-phonylalanyl-allo-threonyl-asparaginyl-serine C-1.7-O-3.1 lactone, 1-Methylindol-3-ylcarbonyl-[4(R)-hydroxy]-L-prolyl-[3-(2-naphthyl)]-L-alanine N-benzyl-N-methylamide, (+)-(2S,3S)-3-[2-Methoxy-5-(trifluoromethoxy)benzylamino]-2-phenylpiperidine, (2R,4S)-N-[1-[3,5-Bis(trifluoromethyl)benzoyl]-2-(4-chlorobenzyl)piperidin-4-yl]quinoline-4-carboxamide, 3-[2(R)-[1 (R)-[3,5-Bis(trifluoromethyl)phenyl]ethoxy]-3(S)-(4-fluorophenyl)morpholin-4-ylmethyl]-5-oxo-4,5-dihydro-1H-1,2,4-triazole-1-phosphlnic acid bis(N-methyl-D-glucamine) salt; [3-[2(R)-[1(R)-[3,5-bis(trifluoromethyl)phenyl]ethoxy]-3(S)-(4-fluorophenyl)-4-morpholinylmethyl]-2,5-dihydro-5-oxo-1H-1,2,4-triazol-1-yl]phosphonic acid 1-deoxy-1-(methylamino)-D-glucitol (1:2) salt, 1'-[2-[2(R)-(3,4-Dichlorophenyl)-4-(3,4,5-trimethoxybenzoyl)morpholin-2-yl]ethyl]spiro[benzo[c]thiophen-1(3H)-4'-piperidine] 2(S)-oxide hydrochloride and the compound CS-003 described in Eur Respir J 2003, 22(Suppl. 45): Abst P2664.

The active compounds In the combination, i.e. the M3 antagonist of the invention, the β2-agonist and any other optional active compounds may be administered together in the same pharmaceutical composition or in different compositions intended for separate, simultaneous, concomitant or sequential administration by the same or a different route.

In a preferred embodiment of the invention the active compounds in the combination are administered by inhalation through a common delivery device, wherein they can be formulated in the same or in different pharmaceutical compositions.

In the most preferred embodiment the M3 antagonist of the invention and the β2-agonist are both present in the same pharmaceutical composition and are administered by inhalation through a common delivery device.

In one aspect the invention provides a combination as herein defined characterised in that the active ingredients (a) and (b) form part of a single pharmaceutical composition.

A said pharmaceutical composition can be prepared by mixing and processing an M3 antagonist of the invention, a β2-agonist and optionally other additives and/or carriers by methods known per se.

The active compounds in the combination, i.e. the M3 antagonist of the invention, the of β2-agonist and any other optional active compounds may be administered by any suitable route, depending on the nature of the disorder to be treated, e.g. orally (as syrups, tablets, capsules, lozenges, controlled-release preparations, fast-dissolving preparations, lozenges, etc); topically (as creams, ointments, lotions, nasal sprays or aerosols, etc); by injection (subcutaneous, intradermic, intramuscular, Intravenous, etc.) or by inhalation (as a dry powder, a solution, a dispersion, etc).

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oll liquid emulsion. The active ingredient may also be presented as a bolus, electuary or paste.

A syrup formulation will generally consist of a suspension or solution of the compound or salt in a liquid carrier for example, ethanol, natural, synthetic or semisynthetic oils such as peanut oil and olive oil, glycerine or water with flavouring, sweetener and/or colouring agent.

Where the composition is In the form of a tablet, any pharmaceutical carrier routinely used for preparing solid formulations may be used. Examples of such carriers include celluloses, stearates such as magnesium stearate or stearic acid, talc, gelatine, acacia, starches, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, lubricating, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i.e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in different primary packaging systems (such as capsules and cartridges of for example gelatine or blisters of for example laminated aluminium foil), for use in an inhaler or insufflator.

Packaging of the formulation may be suitable for unit dose or multi-dose delivery. In the case of multi- dose delivery, the formulation can be pre-metered or metered In use. Dry powder inhalers are thus classified into three groups: (a) single dose, (b) multiple unit dose and (c) multi dose devices.

Formulations generally contain a powder mix for inhalation of the compounds of the invention and a suitable powder base (carrier substance) such as lactose or starch. Use of lactose is preferred. Each capsule or cartridge may generally contain between 2µg and 400 µg of each therapeutically active ingredient. Alternatively, the active ingredient (s) may be presented without excipients.

For single dose inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatine capsules for Inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e.g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92109322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose Inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However In practice, they are not always part of the inhaler design. Because of the high number of doses in multi- dose devices, powder adhesion onto the inner walls of the air conduits and the de- agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even stricter.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.

Such atomisers are described, for example, in PCT Patent Application No. WO 91/14468 and international Patent Application No. WO 97/12687, reference here being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogencontaining chlorofluorocarbon or mixtures thereof, particularly hydrofluoroalkanes, e. g. dichlorodifluoromethane, trichlorofluoromethane, dichlorotetra-fluoroethane, especially 1,1,1,2-tetrafluoroethane, 1,1,1,2,3,3, 3-heptafluoro-n-propane or a mixture thereof. Carbon dioxide or other suitable gas may also be used as propellant. The aerosol composition may be free from excipients other than the propellant or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-6µ. Particles having a size above 20µ are generally too large when Inhaled to reach the small airways. To achieve these particle sizes the particles of the active ingredient as produced may be size reduced by conventional means eg by micronisation or supercritical fluid techniques. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose, manitol or glucose is generally employed. The particle size of the excipient will usually be much greater than the Inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, mucoadhesive agents, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

The proportions in which (a) the β2 agonist and (b) the antagonsit of M3 muscarinic receptors may be used according to the invention are variable. Active substances (a) and (b) may possibly be present in the form of their solvates or hydrates. Depending on the choice of the compounds (a) and (b), the weight ratios which may be used within the scope of the present invention vary on the basis of the different molecular weights of the various salt forms. The pharmaceutical combinations according to the invention may contain (a) and (b) generally in a ratio by weight (b):(a) ranging from 1: 5 to 500: 1, preferably from 1: 10 to 400:1.

The weight ratios specified below are based on the compound (b) expressed as 3(R)-(2-Hydroxy-2,2-dithien-2-ylacetoxy)-1-(3-phenoxypropyl)-1-azoniabicyclo[2.2.2]octane bromide and the free bases of the β2 agonists salmeterol and formoterol which are particularly preferred according to the invention.

The pharmaceutical combinations according to the invention may contain (a) and (b) in the case of formoterol, for example, in a ratio by weight (b):(a) ranging from 1: 10 to 300:1, preferably from 1: 5 to 200:1. preferably 1: 3 to 150:1, more preferably from 1:2 to 100:1.

The pharmaceutical compositions according to the invention containing the combinations of (a) and (b) are normally administered so that 3(R)-(2-Hydroxy-2,2-dithien-2-ylacetoxy)-1-(3-phenoxypropyl)-1-azoniabicyclo[2.2.2]octane bromide and formoterol are present together in doses of 5 to 5000 µg, preferably from 10 to 2000 µg, more preferably from 15 to 1000 µg, better still from 20 to 800 µg per single dose.

For example, without restricting the scope of the invention thereto, combinations in which 3(R)-(2-Hydroxy-2,2-dithien-2-ylacetoxy)-1-(3-phenoxypropyl)-1-azoniabicyclo[2.2.2]octane bromide is used as (b) and formoterol fumarate is used as (a), the compositions according to the invention may contain for instance from 20 to 1000 µg of 3(R)-(2-Hydroxy-2,2-dithien-2-ylacetoxy)-1-(3-phenoxypropyl)-1-azoniabicyclo[2.2.2]octane bromide and from 2,5 to 30 µg of formoterol fumarate.

For example, the active substance combinations according to the invention may contain 3(R)-(2-Hydroxy-2,2-dithien-2-ylacetoxy)-1-(3-phenoxypropyl)-1-azoniabicyclo[2.2.2]octane bromide and (a) in the case of salmeterol, in a ratio by weight (b):(a) in the range from about 1: 30 to 400:1, preferably 1: 25 to 200: 1, preferably 1: 20 to 100:1, more preferably from 1:15 to 50: 1.

The pharmaceutical compositions according to the invention containing the combinations of (a) and (b) are usually administered so that 3(R)-(2-Hydroxy-2,2-dithien-2-ylacetoxy)-1-(3-phenoxypropyl)-1-azonlabicyclo[2.2.2]octane bromide and salmeterol are present together in dosages of 5 to 5000 µg, preferably from 10 to 2000µg, more preferably from 15 to 1000µg, even more preferably from 20 to 800µg per single dose.

For example, without restricting the scope of the invention thereto, combinations in which 3(R)-(2-Hydroxy-2,2-dithien-2-ylacetoxy)-1-(3-phenoxypropyl)-1-azoniabicyclo[2.2.2]octane bromide is used as (b) and salmeterol xinafoate is used as (a), the compositions according to the invention may contain for instance from 20 to 1000 µg of 3(R)-(2-Hydroxy-2,2-dithien-2-ylacetoxy)-1-(3-phenoxypropyl)-1-azoniablcyclo[2.2.2]octane bromide and from 15 to 300 µg of salmeterol xinafoate

The aforementioned examples of possible doses applicable for the combinations according to the invention are to be understood as referring to doses per single application. However, these examples are not be understood as excluding the possibility of administering the combinations according to the invention multiple times. Depending on the medical need patients may receive also multiple inhalative applications. As an example patients may receive the combinations according to the invention for instance two or three times (e. g. two or three puffs with a powder inhaler, an MDI etc) in the morning of each treatment day. As the aforementioned dose examples are only to be understood as dose examples per single application (i. e. per puff) multiple application of the combinations according to the invention leads to multiple doses of the aforementioned examples. The application of the compositions according to the invention can be for instance once a day, or depending on the duration of action of the anticholinergic agent twice a day, or once every 2 or 3 days.

Preferably the composition is In unit dosage form, for example a tablet, capsule or metered aerosol dose, so that the patient may administer a single dose.

Each dosage unit contains suitably from 20 µg to 1000 µg and preferably from 50 µg to 300 µg of an M3 antagonist according to the invention or a pharmaceutical acceptable salt thereof and 1 µg to 300 µg, and preferably from 5 µg to 100 µg of a β2-agonist according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active Ingredients are administered once or twice a day.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for Inhalation delivered with the help of inhalers, especially dry powder Inhalers, however, any other form or parenteral or oral application is possible. Here, the application of inhaled compositions embodies the preferred application form, especially in the therapy of obstructive lung diseases or for the treatment of asthma.

The following preparations forms are cited as formulation examples:

### Example 1

| Ingredient | Amount In µg |
|---|---|
| 3(R)-(2-Hydroxy-2,2-dithien-2-ylacetoxy)-1-(3-phenoxypropyl)-1-azoniabicyclo[2.2.2]octane bromide | 100 |
| Formoterol | 10 |
| Lactose | 2.500 |

### Example 2

| Ingredient | Amount in µg |
|---|---|
| 3(R)-(2-Hydroxy-2,2-dithien-2-ylacetoxy)-1-(3-phenoxypropyl)-1-azoniabicyclo[2.2.2]octane bromide | 100 |
| Salmeterol | 25 |
| Lactose | 2.500 |

### Pharmacological activity

The compositions above are specific examples of preferred embodiments of the invention, wherein an M3 antagonist of the invention is combined with a β2-agonist. These new combinations present significant therapeutic advantages with respect to the combinations of M3 antagonists and a β2-agonist already known in the art.

In particular, the combination of an M3 antagonist of the invention with a β2-agonist, such as salmeterol or formoterol, produces significantly and consistently less heart side-effects, such as tachycardia, than a therapeutically equivalent combination of tiotropium bromide with salmeterol or formoterol.

The following comparative examples describe the advantageous properties of combinations comprising the most preferred M3 antagonist of the invention , i.e. 3(R)-(2-Hydroxy-2,2-dithien-2-ylacetoxy)-1-(3-phenoxypropyl)-1-azoniabicyclo[2.2.2]octane bromide.

### Material and Methods

Three male Beagle dogs weighing 16-19 Kg from the "Centre d'Elevage du domaine des Souches" (CEDS, Mezilles, France) were housed in standard conditions of temperature, humidity and light cycles. The animals were fed standard laboratory chow and water ad libitum.

The animals were fasted for some 18 hours with water ad libitum before the experiment. Each dog was taken from its kennel, weighed, and carried to the room where the experiment was performed by means of a sling suit restrainer.

Left cephalic vein was cannulated to administer the test substances, and surface electrocardiograph leads to record the ECGs (and calcuiate heart rate) were attached to the animal.

Each dog received all the treatments (or the vehicle, i.e. saline solution at 0.9 %) with a wash out period of 6 days as a minimum. The combinations or the vehicle were administered in a total volume of 0.5 ml/kg, In 3-min perfusion. The effects on heart rate were assessed and the end of the administration, and every 15 minutes up to 5 hours after the administration by means of a computer-based data acquisition system MP100WSW (Blopac Systems, Inc Santa Barbara, USA) provided with the program AcqKnowledge *III* (version 3.5.3).

### Results

A preliminary experiment was performed to study the effects of 3(R)-(2-Hydroxy-2,2-dithien-2-ylacetoxy)-1-(3-phenoxypropyl)-1-azoniabicyclo[2.2.2]octane bromide (subsequently called compound 1), tiotropium, salmeterol and formoterol on heart rate in order to identify the most appropriate doses (i.e. the ones producing submaximal heart rate increases) to be administered in combination. (data not shown). The doses selected were the following:
- Compound 1:10 and 100 µg/kg
- Tiotropium: 10 µg/kg
- Salmeterol: 3 µg/kg
- Formoterol: 0.3 µg/kg.

The following combinations were studied:
- Compound .1 at 10 µg/kg plus formoterol at 0.3 µg/kg
- Compound 1 at 10 µg/kg plus salmeterol at 3 µg/kg
- Compound 1 at 100 µg/kg plus salmeterol at 3 µg/kg
- Tiotropium at 10 µg/kg plus formoterol at 0.3 µg/kg
- Tiotropium at 10 µg/kg plus salmeterol at 3 µg/kg

For each treatment the maximum increase in heart rate and the time elapsed before this maximal chronotropic; effect declined to 50 % (t_{50%}) were also measured.

**TABLE 1**

| Treatment | Maxim um heart rate (beats/ min) | Duration of effect - t_{50%} (min) |
|---|---|---|
| Compound 1 at 10 µg/kg + Formoterol at 0,3 µg/kg | 166±11 | 40±18 (a) |
| Tiotropium at 10 µg/kg + Formoterol at 0,3 µg/kg | 206±18 | 155±26 |
| Compound 1 at 10 µg/kg + Salmeterol at 3 µg/kg | 157±14 | 25±10 (b) |
| Compound 1 at 100 µg/kg + Salmeterol at 3 µg/kg | 214±25 | 65±18 (c) |
| Tiotropium at 10 µg/kg + Salmeterol at 3 µg/kg | 206±14 | 130±10 |

The statistical analysis using the One-way ANOVA with Newman-Keuls post test of the data summarised in table 1 shows that there are no differences between the maximum effects on heart rate and that the duration of the effect of: (a) is different from tiotropium plus formoterol p<0.01; (b), is different from tiotropium plus salmeterol p<0.01; (c) is different from tiotropium plus salmeterol p<0.05.

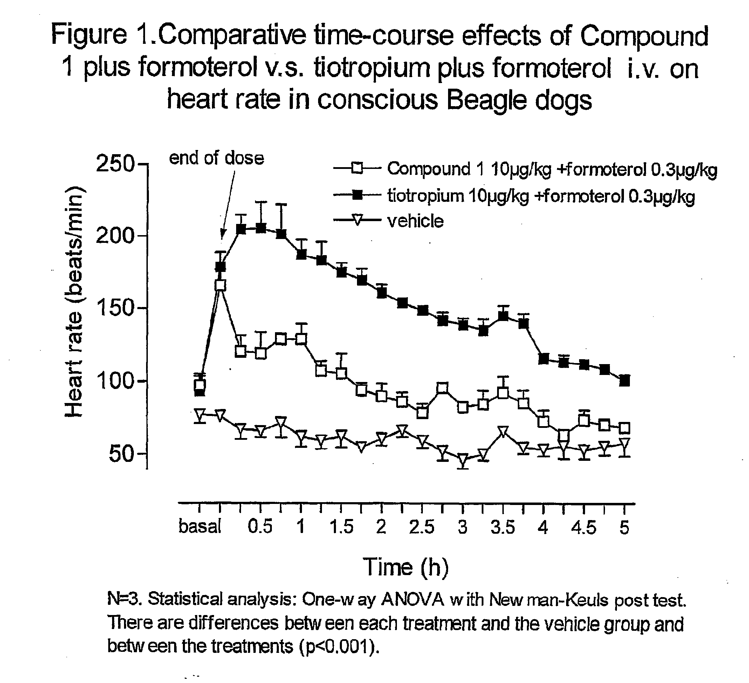

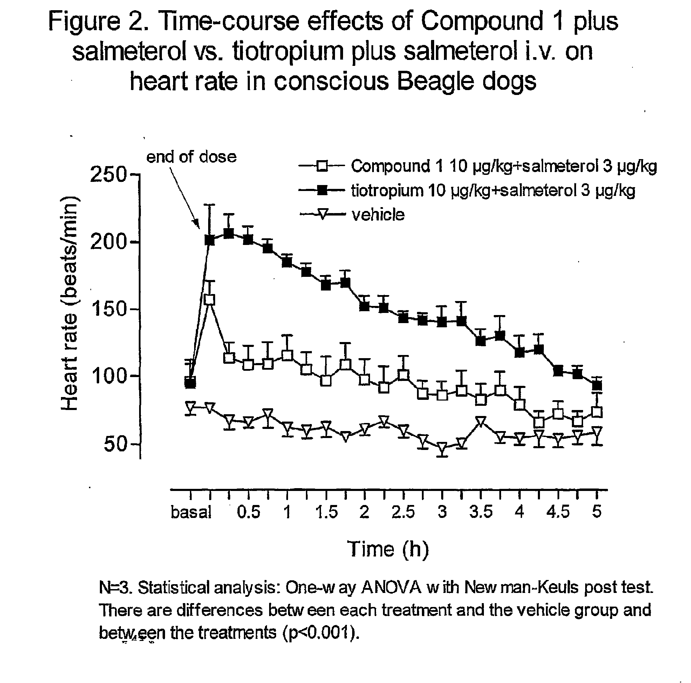

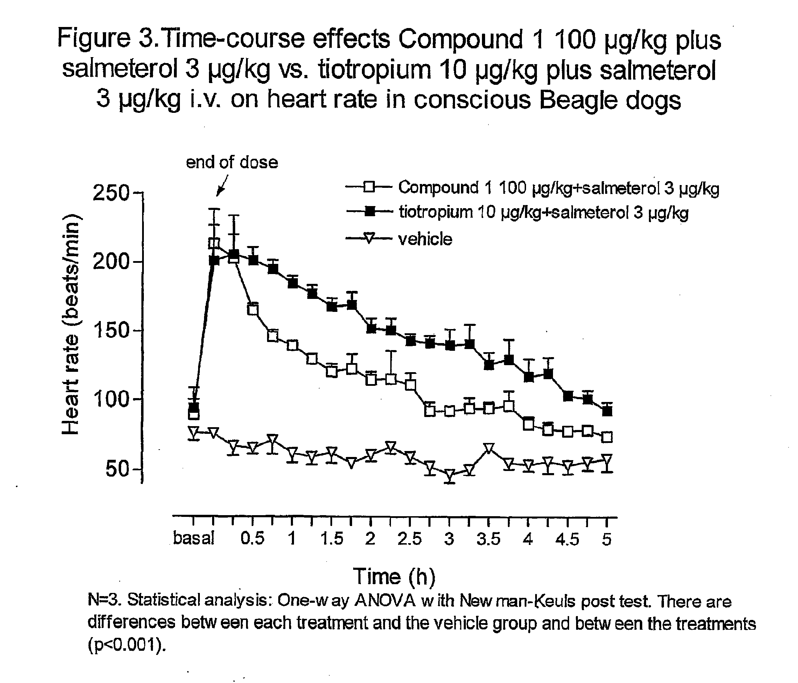

The results summarised in Table 1 and Figures 1 to 3 show the following effects:
The combination of compound 1 (10 µg/kg) plus formoterol produced a smaller increase in heart rate than tiotropium plus formoterol, although the difference is not statistically significant. (Fig. 1)
The chronotropic effects elicited by compound 1 (10 µg/kg) plus formoterol fell to values lower than 50 % of the maximum increase at 40±18 min, whilst tiotropium plus formoterol required 155±26 min to do so. This difference was statistically significant. (Fig. 1)
The combination of compound 1 (10 µg/kg) plus salmeterol also produced a smaller increase in heart rate than tiotropium plus salmeterol. The difference was not statistically significant (Fig. 2).
The chronotropic effects elicited by compound 1 (10 µg/kg) plus salmeterol fell to values lower than 50 % of the maximum increase at 25±10 min, whilst tiotropium plus salmeterol required 130±10 min to do so. This difference was statistically significant (Fig. 2).
The combination of compound 1 at a higher dose (100 µg/kg) plus salmeterol produced a maximum tachycardic effect only slightly greater than the one elicited by the combination of tiotropium at a dose 10-times lower plus salmeterol. This small difference did not attain statistical significance (Fig.3).
The duration of the chronotropic effect produced by a combination of compound 1 at this high dose of 100 µg/kg plus salmeterol is again statistically shorter (t_{50%}=65±18 min) than the one produced by the combination of tiotropium at a dose 10 times smaller (10 µg/kg) plus salmeterol (t_{50%}=130±10 min) (Fig 3).

These results demonstrate that the combination of the M3 antagonists of the invention with LABAs induces less heart side-effects than the combination of commercial M3 antagonists, like tiotropium, with LABAs.

Consequently, the combinations of the invention possess therapeutically advantageous properties, which make them particularly suitable for the treatment of respiratory diseases in all kind of patients, including those having an underlying heart condition.

### BRIEF DESCRIPTION OF DRAWINGS

FIG. 1 shows the time-course effects on heart rate of combinations of 0.3µg/Kg of formoterol with either 10µg/Kg of compound 1 or 10µg/Kg of tiotropium. The effects of a vehicle are also shown as a reference.
FIG. 2 shows the time-course effects on heart rate of combinations of 3µg/Kg of salmeterol with either 10µg/Kg of compound 1 or 10µg/Kg of tiotropium. The effects of a vehicle are also shown as a reference.
FIG. 3 shows the time-course effects on heart rate of combinations of 3µg/Kg of salmeterol with either 100µg/Kg of compound 1 or 10µg/Kg of tiotropium. The effects of a vehicle are also shown as a reference.

## Claims

1. A combination which comprises (a) a β2 agonist and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

2. A combination according to claim 1 wherein the antagonist of M3 muscarinic receptors (b) is 3(R)-(2-hydroxy-2,2-dithien-2-ylacetoxy)-1-(3-phenoxypropyl)-1-azoniabicyclo[2.2.2]octane bromide.

3. A combination according to claim 1 or 2, wherein the β2 agonist is a long-acting β2 agonist.

4. A combination according to any one claims 1 to 3, wherein the β2 agonist is selected from the group comprising arformoterol, bambuterol, bitolterol, broxaterol, carbuterol, clenbuterol, dopexamine, fenoterol, formoterol, hexoprenaline, ibuterol, isoprenaline, levosalbutamol, mabuterol, meluadrine, nolomirole, orciprenaline, pirbuterol, procaterol, (R,R)-formoterol, reproterol, ritodrine, rimoterol, salbutamol, salmeterol, sibenadet, sulfonterol, terbutaline, tulobuterol, GSK-597901, GSK-159797,KUL-1248,TA-2005 and QAB-149, optionally in the form of their racemates, their enantiomers, their diastereomers and mixtures thereof, and optionally their pharmacologically-compatible acid addition salts.

5. A combination according to claim 3 or 4, wherein the β2 agonist is selected from the group comprising formoterol, salmeterol and QAB-149 optionally in the form of their racemates, their enantiomers, their diastereomers and mixtures thereof, and optionally their pharmacologically-compatible acid addition salts.

6. A combination according to claim 5 wherein the β2 agonist is formoterol fumarate.

7. A combination according to claim 5 wherein the β2 agonist is salmeterol xinafoate.

8. A combination according to any one of the preceding claims **characterised in that** the active ingredients (a) and (b) form part of a single pharmaceutical composition.

9. A combination according to any one of the preceding claims, which further comprises (c) another active compound selected from: (i) PDE IV inhibitors, (ii) cortiocosteroids, (iii) leukotriene D4 antagonists, (iv) inhibitors of egfr-kinase, (v) p38 kinase inhibitors and (vi) NK1 receptor agonists.

10. A combination according to claim 9, wherein the active compound (c) is selected from the group consisting of (i) PDE IV inhibitors and (b) cortiocosteroids.

11. Use of (a) a β2-agonist as defined in any one of claims 1 and 3 to 7 and (b) an antagonist of M3 muscarinic receptors as defined in claim 1 or 2, for the preparation of a medicament for simultaneous, concurrent, separate or sequential use in the treatment of a respiratory disease which is asthma, acute or chronic bronchitis, emphysema, chronic obstructive pulmonary disease (COPD), bronchial hyperreactivity or rhinitis, in a patient.

12. Use according to claim 11 wherein the respiratory disease is asthma or chronic obstructive pulmonary disease (COPD).

13. Use according to claim 11 or 12 wherein the patient is suffering from a pre-existing heart condition or condition that would be aggravated by tachycardia.

14. A product comprising (a) a β2 agonist as defined in any one of claims 1 and 3 to 7 and (b) an antagonist of M3 muscarinic receptors as defined in claim 1 or 2, as a combined preparation for simultaneous, concurrent, separate or sequential use in the treatment of a patient suffering from or susceptible to a respiratory disease as defined in claim 11 or 12.

15. A product for use according to claim 14, which further comprises an active compound (c), as defined in claim 9 or 10.

16. A kit of parts comprising (b) an antagonist of M3 muscarinic receptors as defined in claim 1 or 2 together with instructions for simultaneous, concurrent, separate or sequential use in combination with (a) a β2 agonist as defined in any one of claims 1 and 3 to 7 for use in the treatment of a patient suffering from or susceptible to a respiratory disease as defined in claim 11 or 12.

17. A kit for use according to claim 17, which further comprises an active compound (c), as defined in claim 9 or 10.

18. A package comprising (b) an antagonist of M3 muscarinic receptors as defined in claim 1 or 2 and (a) a β2 agonist as defined in any one of claims 1 and 3 to 7, for the simultaneous, concurrent, separate or sequential use in the treatment of a respiratory disease as defined in claim 11 or 12.

19. A package of use according to claim 18, which further comprises an active compound (c), as defined in claim 9 or 10.

20. An antagonist of M3 muscarinic receptors as defined in claim 1 or 2 for simultaneous, concurrent, separate or sequential use in combination with a β2 agonist as defined in any one of claims 1 and 3 to 7, for the treatment of a respiratory disease as defined in claim 11 or 12.

21. An antagonist of M3 muscarinic receptors for use according to claim 20 wherein the patient is suffering from a pre-existing heart condition or condition that would be aggravated by tachycardia

22. A β2 agonist as defined in any one of claims 1 and 3 to 7, for simultaneous, concurrent, separate or sequential use in combination with an antagonist of M3 muscarinic receptors as defined in claim 1 or 2 for the treatment of a respiratory disease as defined in claim 11 or 12.

23. A β2 agonist for use according to claim 22 wherein the patient is suffering from a pre-existing heart condition or condition that would be aggravated by tachycardia.

## Patentansprüche

1. Kombination, die umfasst: (a) einen β2-Agonisten und (b) einen Antagonisten von muskarinischen M3-Rezeptoren, der 3(R)-(2-Hydroxy-2,2-dithien-2-ylacetoxy)-1-(3-phenoxypropyl)-1-azoniabicyclo[2.2.2]octan ist, in der Form eines Salzes, das ein Anion X, welches ein pharmazeutisch verträgliches Anion einer ein- oder mehrwertigen Säure ist, aufweist.

2. Kombination nach Anspruch 1, wobei der Antagonist von muskarinischen M3-Rezeptoren (b) 3(R)-(2-Hydroxy-2,2-dithien-2-ylacetoxy)-1-(3-phenoxypropyl)-1-azoniabicyclo[2.2.2]octan-bromid ist.

3. Kombination nach Anspruch 1 oder 2, wobei der β2-Agonist ein lange wirkender β2-Agonist ist.

4. Kombination nach einem der Ansprüche 1 bis 3, wobei der β2-Agonist ausgewählt ist aus der Gruppe, umfassend Arformoterol, Bambuterol, Bitolterol, Broxaterol, Carbuterol, Clenbuterol, Dopexamin, Fenoterol, Formoterol, Hexoprenalin, Ibuterol, Isoprenalin, Levosalbutamol, Mabuterol, Meluadrin, Nolomirol, Orciprenalin, Pirbuterol, Procaterol, (R,R)-Formoterol, Reproterol, Ritodrin, Rimoterol, Salbutamol, Salmeterol, Sibenadet, Sulfonterol, Terbutalin, Tulobuterol, GSK-597901, GSK-159797, KUL-1248, TA-2005 und QAB-149, gegebenenfalls in der Form von ihren Racematen, ihren Enantiomeren, ihren Diastereomeren und Gemischen davon und gegebenenfalls ihrer pharmakologisch verträglichen Säureadditionssalze.

5. Kombination nach Anspruch 3 oder 4, wobei der β2-Agonist ausgewählt ist aus der Gruppe, umfassend Formoterol, Salmeterol und QAB-149, gegebenenfalls in der Form von ihren Racematen, ihren Enantiomeren, ihren Diastereomeren und Gemischen davon, und gegebenenfalls ihrer pharmakologisch verträglichen Säureadditionssalze.

6. Kombination nach Anspruch 5, wobei der β2-Agonist Formoterolfumarat ist.

7. Kombination nach Anspruch 5, wobei der β2-Agonist Salmeterolxinafoat ist.

8. Kombination nach einem der vorstehenden Ansprüche, dadurch **gekennzeichnet**, dass die aktiven Ingredienzien (a) und (b) einen Teil einer einzigen pharmazeutischen Zusammensetzung bilden.

9. Kombination nach einem der vorstehenden Ansprüche, die des Weiteren umfasst: (c) eine weitere aktive Verbindung, ausgewählt aus: (i) PDE-IV-Inhibitoren, (ii) Corticosteroiden, (iii) Leukotrien-D4-Antagonisten, (iv) Inhibitoren von egfr-Kinase, (v) p38-Kinase-Inhibitoren und (vi) NK1-Rezeptor-Agonisten.

10. Kombination nach Anspruch 9, wobei die aktive Verbindung (c) ausgewählt ist aus der Gruppe, bestehend aus (i) PDE-IV-Inhibitoren und (b) Corticosteroiden.

11. Verwendung von (a) einem β2-Agonisten, wie in einem der Ansprüche 1 und 3 bis 7 definiert, und (b) einem Antagonisten von muskarinischen M3-Rezeptoren, wie in Anspruch 1 oder 2 definiert, für die Herstellung eines Medikaments zur simultanen, gleichzeitigen bzw. gleichlaufenden, getrennten oder sequentiellen Verwendung bei der Behandlung einer Atemwegserkrankung, die Asthma, akute oder chronische Bronchitis, Emphysem, chronisch-obstruktive Lungenerkrankung (COPD), Bronchialhyperreaktivität oder Rhinitis ist, bei einem Patienten.

12. Verwendung nach Anspruch 11, wobei die Atemwegserkrankung Asthma oder chronisch-obstruktive Lungenerkrankung (COPD) ist.

13. Verwendung nach Anspruch 11 oder 12, wobei der Patient an einer vorher existierenden Herzerkrankung oder einer Erkrankung, die durch Tachykardie verschlimmert würde, leidet.

14. Produkt, umfassend: (a) einen β2-Agonisten, wie in einem der Ansprüche 1 und 3 bis 7 definiert, und (b) einen Antagonisten von muskarinischen M3-Rezeptoren, wie in Anspruch 1 oder 2 definiert, als Kombinationspräparat für die simultane, gleichzeitige bzw. gleichlaufende, getrennte oder sequentielle Verwendung bei der Behandlung eines Patienten, der an einer Atemwegserkrankung, wie in Anspruch 11 oder 12 definiert, leidet oder dafür anfällig ist.

15. Produkt zur Verwendung nach Anspruch 14, das außerdem eine aktive Verbindung (c), wie in Anspruch 9 oder 10 definiert, umfasst.

16. Kit von Komponenten, umfassend: (b) einen Antagonisten von muskarinischen M3-Rezeptoren, wie in Anspruch 1 oder 2 definiert, zusammen mit Anweisungen für die simultane, gleichzeitige bzw. gleichlaufende, getrennte oder sequentielle Verwendung in Kombination mit (a) einem β2-Agonisten, wie in einem der Ansprüche 1 und 3 bis 7 definiert, für die Behandlung eines Patienten, der an einer Atemwegserkrankung, wie in Anspruch 11 oder 12 definiert, leidet oder dafür anfällig ist.

17. Kit zur Verwendung nach Anspruch 17, der außerdem eine aktive Verbindung (c), wie in Anspruch 9 oder 10 definiert, umfasst.

18. Packung, umfassend: (b) einen Antagonisten von muskarinischen M3-Rezeptoren, wie in Anspruch 1 oder 2 definiert, und (a) einen β2-Agonisten, wie in einem der Ansprüche 1 und 3 bis 7 definiert, für die simultane, gleichzeitige bzw. gleichlaufende, getrennte oder sequentielle Verwendung bei der Behandlung einer Atemwegserkrankung, wie in Anspruch 11 oder 12 definiert.

19. Packung zur Verwendung nach Anspruch 18, die außerdem eine aktive Verbindung (c), wie in Anspruch 9 oder 10 definiert, umfasst.

20. Antagonist von muskarinischen M3-Rezeptoren, wie in Anspruch 1 oder 2 definiert, für die simultane, gleichzeitige bzw. gleichlaufende, getrennte oder sequentielle Verwendung in Kombination mit einem β2-Agonisten, wie in einem der Ansprüche 1 und 3 bis 7 definiert, für die Behandlung einer Atemwegserkrankung, wie in Anspruch 11 oder 12 definiert.

21. Antagonist von muskarinischen M3-Rezeptoren zur Verwendung nach Anspruch 20, wobei der Patient an einer vorher existierenden Herzerkrankung oder einer Erkrankung, die durch Tachykardie verschlimmert würde, leidet.

22. β2-Agonist, wie in einem der Ansprüche 1 und 3 bis 7 definiert, zur simultanen, gleichzeitigen bzw. gleichlaufenden, getrennten oder sequentiellen Verwendung in Kombination mit einem Antagonisten von muskarinischen M3-Rezeptoren, wie in Anspruch 1 oder 2 definiert, für die Behandlung einer Atemwegserkrankung, wie in Anspruch 11 oder 12 definiert.

23. β2-Agonist für eine Verwendung nach Anspruch 22, wobei der Patient an einer vorher existierenden Herzerkrankung oder einer Erkrankung, die durch Tachykardie verschlimmert würde, leidet.

## Revendications

1. Combinaison qui comprend (a) un agoniste β2 et (b) un antagoniste des récepteurs muscariniques M3 qui est le 3(R)-(2-hydroxy-2,2-dithién-2-ylacétoxy)-1-(3-phénoxypropyl)-1-azoniabicyclo[2.2.2]octane, sous la forme d'un sel ayant un anion X, qui est un anion pharmaceutiquement acceptable d'un acide mono ou polyvalent.

2. Combinaison selon la revendication 1, dans laquelle l'antagoniste des récepteurs muscariniques M3 (b) est le bromure de 3(R)-(2-hydroxy-2,2-dithién-2-ylacétoxy)-1-(3-phénoxypropyl)-1-azoniabicyclo[2.2.2]-octane.

3. Combinaison selon la revendication 1 ou 2, dans laquelle l'agoniste β2 est un agoniste β2 à action prolongée.

4. Combinaison selon l'une quelconque des revendications 1 à 3, dans laquelle l'agoniste β2 est choisi dans le groupe constitué par l'arformotérol, le bambutérol, le bitoltérol, le broxatérol, le carbutérol, le clenbutérol, la dopéxamine, le fénotérol, le formotérol, l'hexoprénaline, l'ibutérol, l'isoprénaline, le lévosalbutamol, le mabutérol, la méluadrine, le nolomirole, l'orciprénaline, le pirbutérol, le procatérol, le (R,R)-formotérol, le réprotérol, la ritodrine, le rimotérol, le salbutamol, le salmétérol, le sibenadet, le sulfontérol, la terbutaline, le tulobutérol, GSK-597901, GSK-159797, KUL-1248, TA-2005 et QAB-149, facultativement sous la forme de leurs racémates, de leurs énantiomères, de leurs diastéréoisomères et de mélanges de ceux-ci, et facultativement leurs sels d'addition avec un acide pharmacologiquement compatibles.

5. Combinaison selon la revendication 3 ou 4, dans laquelle l'agoniste β2 est choisi dans le groupe constitué par le formotérol, le salmétérol et QAB-149 facultativement sous la forme de leurs racémates, de leurs énantiomères, de leurs diastéréoisomères et de mélanges de ceux-ci, et facultativement leurs sels d'addition avec un acide pharmacologiquement compatibles.

6. Combinaison selon la revendication 5, dans laquelle l'agoniste β2 est le fumarate de formotérol.

7. Combinaison selon la revendication 5, dans laquelle l'agoniste β2 est le salmétérol xinafoate.

8. Combinaison selon l'une quelconque des revendications précédentes, **caractérisée en ce que** les ingrédients actifs (a) et (b) font partie d'une composition pharmaceutique unique.

9. Combinaison selon l'une quelconque des revendications précédentes, qui comprend en outre (c) un autre composé actif choisi parmi : (i) les inhibiteurs des PDE IV, (ii) les corticostéroïdes, (iii) les antagonistes du leukotriène D4, (iv) les inhibiteurs d'EGFR-kinase, (v) les inhibiteurs de p38 kinase et (vi) les agonistes du récepteur NK1.

10. Combinaison selon la revendication 9, dans laquelle le composé actif (c) est choisi dans le groupe constitué par (i) les inhibiteurs des PDE IV et (ii) les corticostéroïdes.

11. Utilisation de (a) un agoniste β2 tel que défini dans l'une quelconque des revendications 1 et 3 à 7 et de (b) un antagoniste des récepteurs muscariniques M3 tel que défini dans la revendication 1 ou 2, pour la préparation d'un médicament pour une utilisation simultanée, concomitante, séparée ou successive dans le traitement d'une maladie respiratoire qui est l'asthme, une bronchite aiguë ou chronique, un emphysème, une bronchopneumopathie chronique obstructive (BPCO), une hyperréactivité bronchique ou une rhinite, chez un patient.

12. Utilisation selon la revendication 11, dans laquelle la maladie respiratoire est l'asthme ou une bronchopneumopathie chronique obstructive (BPCO).

13. Utilisation selon la revendication 11 ou 12, dans laquelle le patient est atteint d'une pathologie cardiaque pré-existante ou d'une pathologie qui serait aggravée par tachycardie.

14. Produit comprenant (a) un agoniste β2 tel que défini dans l'une quelconque des revendications 1 et 3 à 7 et (b) un antagoniste des récepteurs muscariniques M3 tel que défini dans la revendication 1 ou 2, sous la forme d'une préparation combinée pour une utilisation simultanée, concomitante, séparée ou successive dans le traitement d'un patient souffrant de ou sensible à une maladie respiratoire telle que définie dans la revendication 11 ou 12.

15. Produit pour une utilisation selon la revendication 14, qui comprend en outre un composé actif (c) tel que défini dans la revendication 9 ou 10.

16. Kit de pièces comprenant (b) un antagoniste des récepteurs muscariniques M3 tel que défini dans la revendication 1 ou 2, conjointement avec des instructions pour une utilisation simultanée, concomitante, séparée ou successive, en combinaison avec (a) un agoniste β2 tel que défini dans l'une quelconque des revendications 1 et 3 à 7 pour une utilisation dans le traitement d'un patient souffrant de ou sensible à une maladie respiratoire telle que définie dans la revendication 11 ou 12.

17. Kit pour une utilisation selon la revendication 16, qui comprend en outre un composé actif (c) tel que défini dans la revendication 9 ou 10.

18. Conditionnement comprenant (b) un antagoniste des récepteurs muscariniques M3 tel que défini dans la revendication 1 ou 2 et (a) un agoniste β2 tel que défini dans l'une quelconque des revendications 1 et 3 à 7, pour une utilisation simultanée, concomitante, séparée ou successive dans le traitement d'une maladie respiratoire telle que définie dans la revendication 11 ou 12.

19. Conditionnement selon la revendication 18, qui comprend en outre un composé actif (c) tel que défini dans la revendication 9 ou 10.

20. Antagoniste des récepteurs muscariniques M3 tel que défini dans la revendication 1 ou 2 pour une utilisation simultanée, concomitante, séparée ou successive en combinaison avec (a) un agoniste β2 tel que défini dans l'une quelconque des revendications 1 et 3 à 7, pour le traitement d'une maladie respiratoire telle que définie dans la revendication 11 ou 12.

21. Antagoniste des récepteurs muscariniques M3 pour une utilisation selon la revendication 20, dans lequel le patient est atteint d'une pathologie cardiaque pré-existante ou d'une pathologie qui serait aggravée par tachycardie.

22. Agoniste β2 tel que défini dans l'une quelconque des revendications 1 et 3 à 7, pour une utilisation simultanée, concomitante, séparée ou successive en combinaison avec un antagoniste des récepteurs muscariniques M3 tel que défini dans la revendication 1 ou 2 pour le traitement d'une maladie respiratoire telle que définie dans la revendication 11 ou 12.

23. Agoniste β2 pour une utilisation selon la revendication 22, dans lequel le patient est atteint d'une pathologie cardiaque pré-existante ou d'une pathologie qui serait aggravée par tachycardie.
